(19) Europäisches Patentamt
European Patent Office
Office européen des brevets

(11) **EP 2 252 708 B1**

(12) **EUROPEAN PATENT SPECIFICATION**

(45) Date of publication and mention of the grant of the patent:
**06.11.2013 Bulletin 2013/45**

(21) Application number: **09723032.0**

(22) Date of filing: **19.03.2009**

(51) Int Cl.:
***C12Q 1/68*** *(2006.01)*

(86) International application number:
**PCT/US2009/037593**

(87) International publication number:
**WO 2009/117537 (24.09.2009 Gazette 2009/39)**

(54) **NON-COMPETITIVE INTERNAL CONTROLS FOR USE IN NUCLEIC ACID TESTS**

NICHT KOMPETITIVE INTERNE STEUERUNGEN ZUR VERWENDUNG IN NUKLEINSÄURETESTS

CONTRÔLE INTERNES NON COMPÉTITIFS DESTINÉS À ÊTRE UTILISÉS DANS DES ESSAIS D'ACIDE NUCLÉIQUE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **20.03.2008 US 38064 P**
**19.05.2008 US 54191 P**

(43) Date of publication of application:
**24.11.2010 Bulletin 2010/47**

(73) Proprietor: **Siemens Healthcare Diagnostics Inc.**
**Deerfield, IL 60015-0778 (US)**

(72) Inventors:
- **DETMER, Jill**
**Kensington, CA 94707 (US)**
- **JIANG, Xiaoqiao**
**Concord, CA 94518 (US)**
- **LE, Minh**
**El Cerrito, CA 94530 (US)**
- **SHERMAN, David**
**Davis, CA 95618 (US)**

(74) Representative: **Maier, Daniel Oliver**
**Siemens AG**
**Postfach 22 16 34**
**80506 München (DE)**

(56) References cited:
**US-A1- 2001 029 014 US-A1- 2006 112 453**
**US-A1- 2006 257 860**

- **HARTMAN L J ET AL: "Development of a novel internal positive control for Taqman<(>R) based assays", MOLECULAR AND CELLULAR PROBES, ACADEMIC PRESS, LONDON, GB, vol. 19, no. 1, 1 February 2005 (2005-02-01), pages 51-59, XP004706569, ISSN: 0890-8508, DOI: DOI: 10.1016/J.MCP.2004.07.006**
- **BEHETS J ET AL: "Development and evaluation of a Taqman duplex real-time PCR quantification method for reliable enumeration of Legionella pneumophila in water samples", JOURNAL OF MICROBIOLOGICAL METHODS, ELSEVIER, AMSTERDAM, NL, vol. 68, no. 1, 1 January 2007 (2007-01-01), pages 137-144, XP022731911, ISSN: 0167-7012, DOI: DOI:10.1016/J.MIMET. 2006.07.002 [retrieved on 2007-01-10]**
- **TANG ET AL.: 'A realtime HIV-1 viral load assay for automated quantitation of HIV-1 RNA in genetically diverse group M subtypes A-H, group O and group N samples' JOUMAL OF VIROLOGICAL METHODS vol. 146, 2007, pages 236 - 245**
- **SMITH ET AL.: 'Complete genome sequence of Methanobacterium thermoautotrophicum deltaH: functional analysis and comparative genomics.' JOUMAL OF BACTERIOLOGY vol. 179, no. 22, November 1997, pages 7135 - 7155**
- **DATABASE GENBANK [Online] January 2005 NCBI, XP003025517 Database accession no. BV217527**
- **DATABASE GENBANK [Online] September 2003 NCBI, XP003025518 Database accession no. CD165028**
- **DATABASE GENBANK [Online] July 2003 NCBI, XP003025519 Database accession no. CC841652**

EP 2 252 708 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE GENBANK [Online] December 2007 NCBI, XP003025520 Database accession no. FC052757
- DATABASE BIOSIS [Online] BIOSCIENCES INFORMATION SERVICE, PHILADELPHIA, PA, US September 1999 TERSTEEGEN ADRIAN ET AL: 'Methanobacterium thermoautotrophicum encodes two multisubunit membrane-bound (NiFe) hydrogenases: Transcription of the operons and sequence analysis of the deduced proteins', XP055037896 Database accession no. PREV199900466468

SEQUENCE LISTING

<110> Siemens Healthcare Diagnostics Inc.
Detmer, Jill
Jiang, Xiaoqiao
Le, Minh
Sherman, David

<120> Non-Competitive Internal Controls for Use in Nucleic Acid Tests

<130> 2007P22920WO

<140> 61038064
<141> 2008-03-20

<160> 44

<170> PatentIn version 3.5

<210> 1
<211> 20
<212> DNA
<213> Homo sapiens

<400> 1
taatacgagt cactataggg 20

<210> 2
<211> 21
<212> DNA
<213> Homo sapiens

<400> 2
taatacgagt cactataggg a 21

<210> 3
<211> 32
<212> DNA
<213> Homo sapiens

<400> 3
agtagtctcg agcatgtgca gggatcctga ca 32

<210> 4

<211> 35
<212> DNA
<213> Homo sapiens

<400> 4
tcgtcgacta gttcaccgag cacctccttc aggct 35

<210> 5
<211> 21
<212> DNA
<213> Homo sapiens

<400> 5
gccgccataa gagccataga a 21

<210> 6
<211> 23
<212> DNA
<213> Homo sapiens

<400> 6
gggtaatttg tctctggctt tga 23

<210> 7
<211> 24
<212> DNA
<213> Homo sapiens

<400> 7
cgccctttga tatctgctcc gcag 24

<210> 8
<211> 89
<212> DNA
<213> Homo sapiens

<400> 8

gccgccataa gagccataga ggaggttgag ggtgttgtga cgccctttga tatctgctcc 60

gcagcatcaa agccagagac aaattaccc 89

<210> 9
<211> 22
<212> DNA
<213> Homo sapiens

<400> 9
agaggaggtt gagggtgttg tg 22

<210> 10
<211> 81
<212> DNA
<213> Homo sapiens

<400> 10

agagccatag aggaggttga gggtgttgtg acgccctttg atatctgctc cgcagcatca 60

aagccagaga caaattaccc c 81

<210> 11
<211> 23
<212> DNA
<213> Homo sapiens

<400> 11
tagaggaggt tgagggtgtt gtg 23

<210> 12
<211> 81
<212> DNA
<213> Homo sapiens

<400> 12

tagagccata gaggaggttg agggtgttgt gacgcccttt gatatctgct ccgcagcatc 60

aaagccagag acaaattacc c 81

<210> 13
<211> 24
<212> DNA
<213> Homo sapiens

<400> 13
atagaggagg ttgagggtgt tgtg 24

<210> 14
<211> 82
<212> DNA
<213> Homo sapiens

<400> 14

atagagccat agaggaggtt gagggtgttg tgacgccctt tgatatctgc tccgcagcat 60

caaagccaga gacaaattac cc 82

<210> 15
<211> 24
<212> DNA
<213> Homo sapiens

<400> 15
atagaggagg ttgagggtgt tgtg 24

<210> 16
<211> 19
<212> DNA
<213> Homo sapiens

<400> 16
cottcaggct cgggcagta 19

<210> 17
<211> 21
<212> DNA
<213> Homo sapiens

<400> 17
ccctttgaga tctgctccgc a 21

<210> 18
<211> 121
<212> DNA
<213> Homo sapiens

<400> 18

atagaggagg ttgagggtgt tgtgacgccc tttgagagct gctccgcagc atcaaagcca 60

gagacaaatt acccctggat aggccccacc acgaaccacc cctactgccc gagcctgaag 120

g 121

<210> 19
<211> 20
<212> DNA
<213> Homo sapiens

<400> 19
aattgggccc tctagatgca 20

<210> 20
<211> 22
<212> DNA
<213> Homo sapiens

<400> 20
gatatcaaag ggcgtcacaa ca 22

<210> 21
<211> 23
<212> DNA
<213> Homo sapiens

<400> 21
cagggccgcc ataagagcca tag 23

<210> 22
<211> 122
<212> DNA
<213> Homo sapiens

<400> 22

aattgggccc tctagatgca tgctcgagca tgtgcaggga tcctgacacg gtactggagg 60

caggcagggc cgccataaga gccatagagg aggttgaggg tgttgtgacg ccctttgaga 120

tc 122

<210> 23
<211> 22
<212> DNA
<213> Homo sapiens

<400> 23
ttgtgacgcc ctttgatatc tg 22

<210> 24
<211> 23
<212> DNA
<213> Homo sapiens

<400> 24
ctggataggc cccaccacga acc 23

<210> 25
<211> 24
<212> DNA
<213> Homo sapiens

<400> 25
tccgcagcat caaagccaga gaca 24

<210> 26
<211> 102
<212> DNA
<213> Homo sapiens

<400> 26

ttgtgacgcc ctttgatatc tgctccgcag catcaaagcc agagacaaat tacccctgga 60

taggccccac cacgaaccac ccctactgcc cgagcctgaa gg 102

<210> 27
<211> 34
<212> DNA
<213> Homo sapiens

<400> 27
agtagtctcg agtaaatagc cctcacccac caac 34

<210> 28
<211> 29
<212> DNA
<213> Homo sapiens

<400> 28
tcgtcgacta gtccgagagc gcaggcttc 29

<210> 29
<211> 21
<212> DNA
<213> Homo sapiens

<400> 29
aatagccctc acccaccaac t 21

<210> 30
<211> 20
<212> DNA
<213> Homo sapiens

<400> 30
tccaacggct ctgtgtcaga 20

<210> 31
<211> 22
<212> DNA
<213> Homo sapiens

<400> 31
ccgttacagg caagttactg cg 22

<210> 32
<211> 150
<212> DNA
<213> Homo sapiens

<220>
<221> misc_feature
<222> (119)..(119)
<223> n is a, c, g, or t

<400> 32

aatagccctc acccaccaac tagccgttac aggcaagtta ctgcgcgatg gcgcaccgga 60

cagtccggtg cgccaccggt gcgccaccgg tgcgccaccg gtgcgccaac ggtcacttnc 120

aacggctagt tctgacacag agccgttgga 150

<210> 33
<211> 22
<212> DNA
<213> Homo sapiens

<400> 33
aaatagccct cacccaccaa ct 22

<210> 34
<211> 151
<212> DNA
<213> Homo sapiens

<220>
<221> misc_feature

<222> (120)..(120)
<223> n is a, c, g, or t

<400> 34

aaatagccct cacccaccaa ctagccgtta caggcaagtt actgcgcgat ggcgcaccgg 60

acagtccggt gcgccaccgg tgcgccaccg gtgcgccacc ggtgcgccaa cggtcacttn 120

caacggctag ttctgacaca gagccgttgg a 151

<210> 35
<211> 21
<212> DNA
<213> Homo sapiens

<400> 35
gtccaacggc tctgtgtcag a 21

<210> 36
<211> 151
<212> DNA
<213> Homo sapiens

<220>
<221> misc_feature
<222> (59)..(59)
<223> n is a, c, g, or t

<220>
<221> misc_feature
<222> (119)..(119)
<223> n is a, c, g, or t

<400> 36

aatagccctc acccaccaac tagccgttac aggcaagtta ctgcgcgatg ggtcacttnc 60

cagtccggtg cgccaccggt gcgccaccgg tgcgccaccg gtgcgccaac ggtcacttnc 120

aacggctagt tctgacacag agccgttgga c 151

<210> 37
<211> 426
<212> DNA
<213> Homo sapiens

<400> 37

agtagtctcg agcatgtgca gggatcctga cacggtactg tcatcagagc tcgtacacgt 60

ccctaggact gtgccatgac gaggcaggca gggccgccat aagagccata gaggaggttg 120

ctccgtccgt cccggcggta ttctcggtat ctcctccaac agggtgttgt gacgcccttt 180

gatatctgct ccgcagcatc tcccacaaca ctgcgggaaa ctatagacga ggcgtcgtag 240

aaagccagag acaaattacc cctggatagg ccccaccacg tttcggtctc tgtttaatgg 300

ggacctatcc ggggtggtgc aaccacccct actgcccgag cctgaaggag gtgctcggtg 360

ttggtgggga tgacgggctc ggacttcctc cacgagccac aactagtcga cgattgatca 420

gctgct 426

<210> 38
<211> 195
<212> DNA
<213> Homo sapiens

<400> 38

tcgagcatgt gcagggatcc tgacacggta ctggaggcag gcagggccgc cataagagcc 60

atagaggagg ttgagggtgt tgtgacgccc tttgatatct gctccgcagc atcaaagcca 120

gagacaaatt acccctggat aggccccacc acgaaccacc cctactgccc gagcctgaag 180

gaggtgctcg gtgaa 195

<210> 39
<211> 247
<212> DNA
<213> Homo sapiens

<400> 39

gggcgaattg ggccctctag atgcatgctc gagcatgtgc agggatcctg acacggtact 60

ggaggcaggc agggccgcca taagagccat agaggaggtt gagggtgttg tgacgccctt 120

tgatatctgc tccgcagcat caaagccaga gacaaattac ccctggatag gccccaccac 180

gaaccacccc tactgcccga gcctgaagga ggtgctcggt gaactagtgg atccgagctc 240

ggtacca 247

<210> 40
<211> 247
<212> RNA
<213> Homo sapiens

<400> 40

gggcgaauug ggcccucuag augcaugcuc gagcaugugc agggauccug acacgguacu 60

ggaggcaggc agggccgcca uaagagccau agaggagguu gagggguguug ugacgcccuu 120

ugauaucugc uccgcagcau caaagccaga gacaaauuac cccuggauag gccccaccac 180

gaaccacccc uacugcccga gccugaagga ggugcucggu gaacuagugg auccgagcuc 240

gguacca 247

<210> 41
<211> 568
<212> DNA
<213> Homo sapiens

<220>
<221> misc feature
<222> (253)..(253)
<223> n is a, c, g, or t

<220>
<221> misc_feature
<222> (293)..(293)
<223> n is a, c, g, or t

<400> 41

agtagtctcg agtaaatagc cctcacccac caactagccg tcatcagagc tcatttatcg 60

ggagtgggtg gttgatcggc ttacaggcaa gttactgcgc gatggcgcac cggacagtcc 120

aatgtccgtt caatgacgcg ctaccgcgtg gcctgtcagg ggtgcgccac cggtgcgcca 180

ccggtgcgcc accggtgcgc ccacgcggtc gccacgcggt ggccacgcgg tggccacgcg 240

caacggtcac ttncaacggc tagttctgac acagagccgt gttgccagtg aangttgccg 300

atcaagactg tgtctcggca tggactcatg acgcaccgga cagtgaatag ttcactgtcc 360

acctgagtac tgcgtggcct gtcacttatc aagtgacagg ggtgcacacc ggacagtccg 420

gtgcggtgtc cggtgtgcca ccacgtgtgg cctgtcaggc cacgccacag gccacacggt 480

ctaaaattca tctccgaagc ctgcgctctc ggactagtcg gattttaagt agaggcttcg 540

gacgcgagag cctgatcagc acgatgct 568

<210> 42
<211> 266
<212> DNA
<213> Homo sapiens

<400> 42

tcgagtaaat agccctcacc caccaactag ccgttacagg caagttactg cgvgatggcg 60

caccggacag tccggtgcgc caccggtgcg ccaccggtgc gccaccggtg cgccaacggt 120

cacttccaac ggctagttct gacacagagc cgttggactc atgacgcacc ggacagtgaa 180

tagttcactg tccggtgcac accggacagt ccggtgcggt gtccggtgtg ccactaaaat 240

tcatctccga agcctgcgct ctcgga 266

<210> 43
<211> 318
<212> DNA
<213> Homo sapiens

<220>
<221> misc_feature
<222> (154)..(154)
<223> n is a, c, g, or t

<400> 43

gggcgaattg ggccctctag atgcatgctc gagtaaatag ccctcaccca ccaactagcc 60

gttacaggca agttactgcg cgatggcgca ccggacagtc cggtgcgcca ccggtgcgcc 120

accggtgcgc caccggtgcg ccaacggtca cttncaacgg ctagttctga cacagagccg 180

ttggactcat gacgcaccgg acagtgaata gttcactgtc cggtgcacac cggacagtcc 240

ggtgcggtgt ccggtgtgcc actaaaattc atctccgaag cctgcgctct cggactagtg 300

catccgagct cggtacca 318

<210> 44
<211> 318
<212> RNA
<213> Homo sapiens

<220>
<221> misc_feature
<222> (154)..(154)
<223> n is a, c, g, or u

<400> 44

gggcgaauug ggcccucuag augcaugcuc gaguaaauag cccucaccca ccaacuagcc 60

guuacaggca aguuacugcg cgauggcgca ccggacaguc cggugcgcca ccggugcgcc 120

accggugcgc caccggugcg ccaacgguca cuuncaacgg cuaguucuga cacagagccg 180

uuggacucau gacgcaccgg acagugaaua guucacuguc cggugcacac cggacagucc 240

ggugcggugu ccggugugcc acuaaaauuc aucuccgaag ccugcgcucu cggacuagug 300

cauccgagcu cgguacca 318

## Description

### TECHNICAL FIELD

This application relates generally to tools for conducting diagnostic assays and more specifically to internal control sequences for use in nucleic acid tests (NATs) that do not compete with the target nucleic acid sequences.

### BACKGROUND OF THE INVENTION

In order to ensure that nucleic acid tests (NATs) are properly performed, the assays require the presence of internal controls. In diagnostic NATs, the presence of an internal control can guarantee the integrity of the test. Specifically, by including an internal control in a NAT, samples testing positive for the internal control and the target nucleic acid are true positives. By contrast, samples testing only for the internal control are true negatives, samples testing only for the target nucleic acid are true negatives, and samples having no detectable internal control or target are false negatives.

The most commonly used diagnostic assay requiring the presence of internal controls is the polymerase chain reaction (PCR) assay, which is a target amplification assay in its traditional use and a target amplification and quantification assay in its modified use. There are several types of PCR assays: traditional PCR (amplification of DNA); reverse-transcriptase PCR (also known as "RT-PCR"; amplification of DNA using RNA as a starting material), real-time PCR (simultaneous quantification and amplification of DNA); and real-time RT-PCR (simultaneous quantification and amplification of DNA using RNA as a starting material).

In amplification assays, such as PCR assays, typically, one of two types of internal controls is used: competitive internal controls and non-competitive internal controls.

Hartman et al an exogenous internal positive contral (IPC) based on Taqman(R) Chemistry which is not from M. thermoautotraphicum (HARTMAN L J ET AL: "Oevelopment of a novel internal positive control for Taqman (R) based assays", MOLECULAR ANO CELLULAR PROBES, ACAOEMIC PRESS, LONOON, GB, vol. 19, no. 1, 1 February 2005 (2005-02-01), pages 51-59).

With competitive internal controls, the target and the internal control are amplified with one common set of primers under the same conditions and in the same PCR tube. With competitive internal controls, the internal control nucleic acid is flanked by the same primer sequence that is used to initiate amplification of the target nucleic acid. When the PCR assay is performed correctly, the IC nucleic acid will be detected during post amplification analysis. As is clear from its name, competitive internal controls are based on competition between target DNA and the internal control. For competitive internal controls to be effective, the amount of internal control in the sample tube is critical to the detection limit. A disadvantage of the use of competitive internal controls is based upon its structure, specifically, simultaneous amplification of two different nucleic acid fragments flanked by the same primer sites risks inhibition or enhancement of one or both products depending on the molar ratio, the length, the sequence, and the secondary structure of the nucleic acid fragments. Another disadvantage of competitive internal controls is that they are incapable for use in multiplex assays, which screen multiple targets in a single assay.

With non-competitive internal controls, the target and the internal control are amplified using a different primer set for each; thus, non-competitive internal controls require a PCR in which two reactions with different kinetics proceed simultaneously. Because the kinetics of the two reactions is different, there is no competition for the primers. Non-competitive internal control primer sets currently in use typically target genes other than the target gene (e.g., encoding rRNA), which are present in a sample in higher copy number than the target gene. The most commonly used non-competitive internal control in the art uses primers specific to conserved sequences of 16S and 23 S ribosomal DNA. Hartman et al (2005) Molecular and Cellular Probes, 19, 51-59 discloses an exogenous internal positive control (IPC) with mutated primer and probe sites. The resulting sequence showed no homology to known published sequence data and is therefore an example of a non-competitive standard. There remains a need in the art for additional non-competitive internal controls that may be prepared for use in multiple assays. An advantage of non-competitive internal controls is that unlike competitive internal controls, non-competitive internal controls they may be stored for use in multiple reactions and also may be used in multiplex reactions. There is a need in the art for such a non-competitive internal control.

### SUMMARY OF THE INVENTION

The present invention overcomes the need in the art for a non-competitive internal control for use in NATs by providing nucleic acid sequences that may be prepared in the lab and stored for use in multiple reactions and in multiplex NATs.

In one aspect of the invention, there is provided the use of a non-competitive internal control for use in nucleic acid tests (NATs) comprising a nucleic acid obtained from an organism selected from *Methanobacterium thermoautrophicum* (MET).

In one embodiment of the invention, the non-competitive internal controls are comprised of DNA and are used in DNA NATs selected from the group consisting of Influenza A, Influenza B, parainfluenza viruses 1 to 4 (PIV-1 to PIV-4), respiratory syncytial virus type A (RSV A), RSV B, human metapneumovirus (hMPV), *Chlamydia trachomatis* (CT), *Neisseria gonorrhea* (GC [for *gonococci]),* and Hepatitis B virus (HBV).

In another embodiment of the invention, the non-competitive internal control are comprised of RNA and are used in RNA NATs selected from the group consisting Hepatitis C virus (HCV), Human Immunodeficiency Virus 1 (HIV-1), and Severe Acute Respiratory Syndrome (SARS).

In another aspect of the invention, there is provided, a method of preparing a non-competitive internal control for use in nucleic acid tests (NATs), comprising the steps of: (a) extracting genomic DNA from *Methanobacterium thermoautrophicum* (MET); (b) generating an amplicon from the genomic DNA of step (a) using forward and reverse primers having at least two restriction enzyme sites and at least one target specific sequence; (c) generating a plasmid by ligating the amplicon of step (b) with a vector sequence having a promoter sequence and restriction enzyme sites that are identical to the restriction enzyme sites of the amplicon; and (d) digesting the plasmid with restriction enzymes corresponding to the restriction enzyme sites of steps (b) and (c) to generate MET internal control DNA.

Where RNA is required, the method further comprises the step of (e): preparing MET internal control RNA from the DNA of step (d).

In one embodiment of the invention, the at least two restriction enzyme sites of steps (b) and (d) (for MET) correspond to the sequences of restriction enzymes XhoI and SpeI.

In another embodiment of the invention, the promoter sequence of step (c) (for MET) is a T7 promoter sequence.

In a further embodiment of the invention, the MET internal control DNA is used as an non-competitive internal control in DNA NATs selected from the group consisting of: Influenza A, Influenza B, parainfluenza viruses 1 to 4 (PIV-1 to PIV-4), respiratory syncytial virus type A (RSV A), RSV B, human metapneumovirus (hMPV), *Chlamydia trachomatis* (CT), *Neisseria gonorrhea* (GC), and Hepatitis B virus (HBV).

In yet another embodiment of the invention MET internal control RNA is used as a non-competitive internal control in RNA NATs selected from the group consisting of: Hepatitis C virus (HCV), Human Immunodeficiency Virus 1 (HIV-1), and Severe Acute Respiratory Syndrome (SARS).

Additional aspects, advantages and features of the invention will be set forth, in part, in the description that follows, and, in part, will become apparent to those skilled in the art upon examination of the following, or may be learned by practice of the invention.

## BRIEF DESCRIPTION OF THE DRAWINGS

**FIG. 1** is a schematic diagram of the method for preparing the internal control sequences of the present invention.

**FIG. 2** is a graph of the amplification plot for *Chlamydia trachomatis* (CT) (left curve) and MET IC (right curve) in a single well.

**FIG. 3** is a graph of the amplification plot for *Neisseria gonorrhea* (GC) (left curve) and MET IC (right curve) in a single well.

**FIG. 4** is a graph of the amplification plot for Hepatitis C virus (HCV) (left curve) and MET IC (right curve) in a single well.

## DETAILED DESCRIPTION OF THE INVENTION

Set forth below is a description of what are currently believed to be the preferred embodiments and best examples of the claimed invention. Any alternates or modifications in function, purpose, or structure are intended to be covered by the claims of this application.

DEFINITIONS:

In describing and claiming the present invention, the following terminology the following definitions are used for the purpose of describing particular embodiments only, and is not intended to be limiting. As used in this specification and the appended claims, the singular forms "a," "an," and "the" include plural referents unless the context clearly dictates otherwise.

The term "non-competitive internal control" refers to an internal control nucleic acid sequence that includes primer sites that are not present in the target nucleic acid sequence.

The term "competitive internal control" refers to an internal control nucleic acid sequence that includes primer sites that are also present in the target nucleic acid sequence.

The terms "FW" and "FP" indicate forward primers and the terms "RV" and "RP" indicate reverse primers. The term "P" when used alone refers to a probe.

The term "PCR primer for the construction of internal control (IC) clone" refers to oligonucleotides that were designed to introduce unique sequences and restriction sites into a newly constructed IC plasmid DNA through overlapping PCR reactions.

The term "amplification primer" (also referred to herein as "primer") refers to an oligonucleotide that is complementary to DNA or RNA molecules and provides the 3 -OH-end of a substrate to which any DNA polymerase can add the nucleotides of a growing DNA chain in the 5 to 3 direction.

The term "detection probe" (also referred to herein as "probe") refers to an oligonucleotide capable of selectively hybridizing to the amplified target nucleic acid under appropriate conditions. The detection probe may consist of a nucleotide with 5 -reporter dye (R) and a 3 -quencher dye (Q). A fluorescent reporter dye and fluorophore or a quencher that is either red-shifted fluorescent or non-fluorescent may be covalently linked to the 5 -end or 3 -end of the oligonucleotide. The detection probe acts as a TAQMAN® (Applied Biosystems, Foster City, CA) probe or other detection probes, such as beacons, non-nuclease real time amplification probes during amplification and detection process.

The term "diagnostic target (unknown)" refers to the nucleic acid sequence(s) that the PCR assay has been designed to detect specifically. Examples of these assays include targets, such as for example, Hepatitis B Virus (HBV), Hepatitis C Virus (HCV), and Human Immunodeficiency Virus (HIV).

The term "diagnostic target (known)" refers to the unique DNA or RNA target that is spiked at a known concentration into either the extraction step or amplification mixture used to isolate and amplify the Specific Diagnostic Target whose presence or quantity in the sample is unknown. In addition, unique primers and probes that recognize the unique fragments of RNA or DNA are added into the amplification mixture. These internal controls can be used to monitor the efficiency of the target extraction, amplification, and detection in real time kPCR assays.

As used herein, the term "target amplification" refers to enzyme-mediated procedures that are capable of producing billions of copies of nucleic acid target. Examples of enzyme-mediated target amplification procedures known in the art include PCR, nucleic acid-sequence-based amplification ("NASBA"), transcription-mediated amplification ("TMA"), strand displacement amplification ("SDA"), and ligase chain reaction ("LCR"). The most widely used target amplification procedure is PCR, first described for the amplification of DNA by Mullins et al. in U.S. Patent No. 4,683,195 and Mullis in U.S. Patent No. 4,683,202. The PCR procedure is well known to those of ordinary skill in the art. Where the starting material for the PCR reaction is RNA, complementary DNA ("cDNA") is made from RNA via reverse transcription. A PCR used to amplify RNA products is referred to as reverse transcriptase PCR or "RT-PCR."

In the PCR technique, a sample of DNA is mixed in a solution with a molar excess of two oligonucleotide primers of 10-30 base pairs each that are prepared to be complementary to the 3' end of each strand of the DNA duplex; a molar excess of unattached nucleotide bases (i.e., dNTPs); and DNA polymerase, (preferably Taq polymerase, which is stable to heat), which catalyzes the formation of DNA from the oligonucleotide primers and dNTPs. Of the two primers, one is a forward primer that will bind in the 5'-3' direction to the 3' end of one strand of the denatured DNA analyte and the other is a reverse primer that will bind in the 3'-5' direction to the 5' end of the other strand of the denatured DNA analyte. The solution is heated to 94-96°C to denature the double-stranded DNA to single-stranded DNA. When the solution cools, the primers bind to the separated strands and the DNA polymerase catalyzes a new strand of analyte by joining the dNTPs to the primers. When the process is repeated and the extension products synthesized from the primers are separated from their complements, each extension product serves as a template for a complementary extension product synthesized from the other primer. In other words, an extension product synthesized from the forward primer, upon separation, would serve as a template for a complementary extension product synthesized from the reverse primer. Similarly, the extension product synthesized from the reverse primer, upon separation, would serve as a template for a complementary extension product synthesized from the forward primer. In this way, the region of DNA between the primers is selectively replicated with each repetition of the process. Since the sequence being amplified doubles after each cycle, a theoretical amplification of one billion copies may be attained after repeating the process for a few hours; accordingly, extremely small quantities of DNA may be amplified using PCR in a relatively short period of time.

As used herein, the term "amplicon" refers to amplified nucleic acid product, such as for example, amplified PCR product.

Where the starting material for the PCR reaction is RNA, complementary DNA ("cDNA") is made from RNA via reverse transcription. The resultant cDNA is then the same positions on the target as the listed sequences. Because it is understood that nucleic acids do not require complete complementarity in order to hybridize, the probe and primer sequences disclosed herein may be modified to some extent without loss of utility as specific primers and probes. Generally, sequences having homology of 80% or more fall within the scope of the present invention.

As used herein, the term "cloning" is used to refer to "molecular cloning," which is a process that creates multiple copies of a nucleic acid sequence (also referred to herein as an "insert"), such as unique genes or selectable genetic markers, from a single copy of the insert. The cloning process typically occurs in a "cloning vector" (also referred to herein as "vector"), which is a DNA molecule, such as a plasmid or viral DNA chromosome, that is capable of replication in a suitable host cell. A "plasmid" is known in the art as a circular double-stranded DNA molecule that is obtained from a bacterial species. A cloning vector typically has one or more suitable sites for the insertion of the nucleic acid sequences. In a successful cloning, the cloning vector is introduced into the host cell and replication of the cloning vector in the host cell results in a transformed host cell, which expresses the nucleic acid sequences that were inserted into the cloning vector. Replication of the cloning vector in the host cell is typically initiated in via a "promoter," which is a regulatory region of DNA located upstream (towards the 5' region) of a gene, and which binds RNA polymerase and transcription factors to initiate RNA transcription. Using this procedure and as shown in FIG. 1, the cloning vector can be used as a template to produce an RNA internal control by routine transcription reaction or a DNA internal control by restriction digestion.

As explained in the Background section, when a non-competitive internal control is used in an amplification reaction, such as PCR, different primer sets are used for the internal control and for the target. The use of the non-competitive internal controls thus requires a PCR in which two reactions with different kinetics proceed simultaneously and the kinetics of each reaction are not influenced by competition for the primers.

An advantage of non-competitive internal controls over competitive internal controls is that because non-competitive internal controls are prepared with their own set of primers, they may be used for many different assays in the same laboratory. Another advantage of non-competitive internal controls is that they can be used for multiplex PCR assays. By contrast, competitive internal controls cannot be used with multiplex PCR assays in which several primer pairs are required. As is known to those of skill in the art, multiplex PCR has much usefulness for molecular diagnostics since multiple pathogens producing similar symptoms may be screened simultaneously in a single reaction.

The non-competitive internal controls of the present invention have at least two primer binding sites and at least one probe binding site. As non-competitive controls, the internal controls of the present invention have unique cloning sequences that do not compete with the target nucleic acid sequences. The internal controls are independently designed from the genomes of the organisms *Methanobacterium thermoautrophicum* (MET). Nucleic acids isolated from the organisms are constructed into a plasmid with a cloning vector.

Procedures for cloning nucleic acids are known to those of skill in the art. FIG. 1 shows an exemplary procedure to clone the internal control nucleic acids of the present invention; the procedure set forth in FIG. 1 was used to generate the non-competitive internal controls described in the Examples. As shown in FIG. 1, the isolated genomic DNA is digested with XhoI and SpeI restriction enzymes and the resulting DNA insert is amplified using PCR and purified. Separately, a vector fragment is prepared from the TOPO® Cloning Vector (Invitrogen, Carlsbad, California), which is digested with XhoI and SpeI restriction enzymes to form a vector fragment with XhoI and SpeI sticky ends, which is subsequently purified. The DNA insert and the vector fragment are then ligated by matching the XhoI and SpeI sticky ends to form a plasmid that includes the genomic DNA insert, the vector fragment, and a T7 promoter sequence. The T7 promoter sequence will typically be either a 20-mer T7 promoter sequence 5'-TAA TAC GAG TCA CTA TAG GG-3' (SEQ ID NO. 1) or a 21-mer T7 promoter sequence 5'-TAA TAC GAG TCA CTA TAG GGA-3' (SEQ ID NO. 2). The internal control DNA sequences of the present invention are generated by digesting the plasmid with XhoI or SpeI. The internal control RNA sequences are obtained by transcribing the DNA under conditions known to those of skill in the art.

Modifications to the procedures set forth in FIG. 1, such as for example, replacing the T7 promoter with other promoters known in the art, such as the T3 and SP6 promoters, or flanking the MET inserts with more than one promoter are within the skill level of those in the art. Similarly, it would be within the skill level of one in the art to modify the method set forth herein by replacing the XhoI and SpeI restriction binding sites with binding sites for other suitable restriction enzymes.

The DNA internal controls of the present invention have utility in DNA nucleic acid tests (DATs), including without limitation: Influenza A, Influenza B, parainfluenza viruses 1 to 4 (PIV-1 to PIV-4), respiratory syncytial virus type A (RSV A), RSV B, human metapneumovirus (hMPV), *Chlamydia trachomatis* (CT), *Neisseria gonorrhea* (GC), and Hepatitis B virus (HBV).

The RNA internal controls of the present invention have utility in RNA nucleic acid tests (NATs), including without limitation: Hepatitis C virus (HCV), Human Immunodeficiency Virus 1 (HIV-1), and Severe Acute Respiratory Syndrome (SARS).

In practice, it is to be understood that the internal controls of the present invention are included in the same reaction mix as the sample that is being targeted. In one embodiment of the invention, the internal controls are introduced at the step of virus lysis and consequently, can be used to monitor the RNA or DNA target capture or release at the sample preparation step and/or to monitor the target amplification and detection during real time PCR.

INTERNAL CONTROL FRAGMENTS AND PRIMER AND PROBE SETS:

A sequence listing describes the (i) DNA insert during cloning; (ii) resulting complete double-stranded DNA sequence based on purification following restriction enzyme digest; and (iii) sequence of the single-stranded RNA generated from the T7 promoter with attached vector sequences.

MET NUCLEIC ACID FRAGMENTS

Table I shows the sequences of the forward (FP) and reverse (RP) primers that are used to extract nucleic acid fragments from the *M. thermoautrophicum* (MET) genome, which are used to clone the MET internal controls (MET IC) of the present invention. The primers are designed with restriction enzymes binding sites (highlighted in bold) and target specific binding sites (underlined). As shown therein, the forward primer is designed with an XhoI restriction enzyme sequence (C/TCGAG) and the reverse primer is designed with a SpeI restriction enzyme sequence (A/CTAGT).

| TABLE 1 | | |
|---|---|---|
| **Fragment Primers** | **Sequence 5 -3** | **Strands** |
| MET IC FP | AGTAGTC**TCGAG**CATGTGCAGGGATCCTGACA (SEQ ID. NO. 3) | (+) |
| MET IC RP | TCGTCGA**CTAGT**TCACCGAGCACCTCCTTCAGGCT (SEQ ID NO. 4) | (-) |

As indicated above, the internal controls of the present invention include at least two primer binding sites and at least one probe binding site. Tables 2 to 8 set forth various forward and reverse amplification primer sequences and detection probe binding sequences that can be used to generate MET IC Amplicons, which are also provided in the tables. The amplicons set forth in Tables 2 to 8 can be used to prepare non-competitive controls for use in nucleic acid diagnostic tests for the following disease states: Influenza A, Influenza B, parainfluenza viruses I to 4 (PIV-1 to PIV-4), respiratory syncytial virus type A (RSV A), RSV B, human metapneumovirus (hMPV), *Chlamydia trachomatis* (CT), *Neisseria gonorrhea* (GC), Hepatitis B virus (HBV), Hepatitis C virus (HCV), Human Immunodeficiency Virus 1 (HIV-1), and Severe Acute Respiratory Syndrome (SARS).

| TABLE 2 | | |
|---|---|---|
| **Set 1** | **Sequence 5 -3** | **Strands** |
| MET-FW01 1 | GCCGCCATAAGAGCCATAGAA (SEQ ID NO. 5) | (+) |
| MET-RV01 1 | GGGTAATTTGTCTCTGGCTTTGA (SEQ ID NO. 6) | (-) |
| MET-P01 | CGCCCTTTGATATCTGCTCCGCAG (SEQ ID NO. 7) | (+) |
| MET IC Amplicon (96 pbs) | 1 GCCGCCATAA GAGCCATAGA GGAGGTTGAG<br>31 GGTGTTGTGA CGCCCTTTGA TATCTGCTCC<br>61 GCAGCATCAA AGCCAGAGAC AAATTACCC<br>(SEQ ID No. 8) | |

| TABLE 3 | | |
|---|---|---|
| **Set 2** | **Sequence 5 -3** | **Strands** |
| MET-FW02 | AGAGGAGGTTGAGGGTGTTGTG (SEQ ID NO. 9) | (+) |
| MET-RV01 | GGGTAATTTGTCTCTGGCTTTGA (SEQ ID NO. 6) | (-) |
| MET-P02 | CGCCCTTTGATATCTGCTCCGCAG (SEQ ID NO. 7) | (+) |
| Set 2 Amplicon (80 bps) | 1 AGAGCCATAG AGGAGGTTGA GGGTGTTGTG<br>31 ACGCCCTTTG ATATCTGCTC CGCAGCATCA<br>61 AAGCCAGAGA CAAATTACCC C<br>(SEQ ID NO. 10) | |

| TABLE 4 | | |
|---|---|---|
| Set 3 | Sequence 5 -3 | Strands |
| MET-FW03 | TAGAGGAGGTTGAGGGTGTTGTG (SEQ ID NO. 11) | (+) |
| MET-RV01 | GGGTAATTTGTCTCTGGCTTTGA | (-) |

| TABLE 8 | | |
|---|---|---|
| Set 7 | Sequence 5□-3□ | Strands |
| Set 7 Amplicon (102 bps) | 1 TTGTGACGCC CTTTGATATC TGCTCCGCAG<br>31 CATCAAAGCC AGAGACAAAT TACCCCTGGA<br>61 TAGGCCCCAC CACGAACCAC CCCTACTGCC<br>91 CGAGCCTGAA GG<br>(SEQ ID NO. 26) | |

It is to be understood that while the invention has been described in conjunction with the preferred specific embodiments thereof, that the foregoing description as well as the examples that follow are intended to illustrate and not limit the scope of the invention. Other aspects, advantages and modifications within the scope of the invention will be apparent to those skilled in the art to which the invention pertains.

All patents and publications mentioned herein are incorporated by reference in their entireties.

**EXPERIMENTAL**

The following examples are put forth so as to provide those of ordinary skill in the art with a complete disclosure and description of how to make and use the compositions of the invention. The examples are intended as non-limiting examples of the invention. While efforts have been made to ensure accuracy with respect to variables such as amounts, temperature, etc., experimental error and deviations should be taken into account. Unless indicated otherwise, parts are parts by weight, temperature is degrees centigrade, and pressure is at or near atmospheric. All components were obtained commercially unless otherwise indicated.

GENERAL PROTOCOLS:

The following protocols, apparatus, and kits were used to carry out the following Examples.

Extraction of Genomic DNA: native target purified by manual Qiagen (Valencia, CA) sample preparation kit.

PCR Conditions and Apparatus: PCR was conducted on an MJ Research (Ramsey, MN) instrument using the following thermoprofile:

95° 10 min – 1 step

95° 15 sec
60° 15 sec } repeated for 30 cycles
68° 1 min

72° 10 min

4° overnight (until removed)

Purification of PCR Product: PCR product was purified using a Qiagen kit (Valencia, CA)

Cloning Procedure: Cloning was carried out using the procedure set forth in the product insert for the Invitrogen cloning protocol using a TOPO® Cloning vector (Carlsbad, CA).

Ligation Procedure: Ligation of DNA fragments to the TOPO® Cloning vector was carried out using the instructions for Invitrogen T4 ligase (Carlsbad, CA).

Purification of Vector Fragment: Purification of vector was carried out using Clontech NUCLEOSPIN® RNA Purification Kit (Mountain View, CA).

RNA Transcription Protocol: Transcription was carried out according to the instructions in the product insert for the Ambion T7 MEGASCRIPT® kit (Austin, TX).

## EXAMPLE 7

### CORN IC DNA TRANSCRIPT SEQUENCE

A plasmid was prepared by ligating the purified MET Corn IC DNA insert sequence of Example 6 to a purified vector fragment with sticky end restriction sites and a T7 promoter sequence. The purified vector fragment was isolated from a TOPO® Cloning vector (Invitrogen, Carlsbad, California) via digestion with the restriction enzymes XhoI and SpeI. The plasmid was formed by matching the XhoI and SpeI sticky ends of the DNA insert and the vector. FIG 1 shows a schematic of the cloning process.

The resultant plasmid was linearized with XhoI and SpeI to generate the following 318 bp Corn IC DNA transcript sequence. The vector sequences are highlighted with bold underlining (SEQ ID NO. 43):

| | | | | |
|---|---|---|---|---|
| 1 | **GGGCGAATTG** | **GGCCCTCTAG** | **ATGCATG**CTC | GAGTAAATAG |
| 41 | CCCTCACCCA | CCAACTAGCC | GTTACAGGCA | AGTTACTGCG |
| 81 | CGATGGCGCA | CCGGACAGTC | CGGTGCGCCA | CCGGTGCGCC |
| 121 | ACCGGTGCGC | CACCGGTGCG | CCAACGGTCA | CTTNCAACGG |
| 161 | CTAGTTCTGA | CACAGAGCCG | TTGGACTCAT | GACGCACCGG |
| 201 | ACAGTGAATA | GTTCACTGTC | CGGTGCACAC | CGGACAGTCC |
| 241 | GGTGCGGTGT | CCGGTGTGCC | ACTAAAATTC | ATCTCCGAAG |
| 281 | CCTGCGCTCT | CGGACTAGT**G** | **CATCCGAGCT** | **CGGTACCA** |

## EXAMPLE 8

### CORN IC RNA TRANSCRIPT SEQUENCE

The following sequence is the 318 bp Corn IC RNA transcript sequence prepared from the DNA sequence of Example 7. The vector sequences are highlighted with bold underlining (SEQ ID NO. 44):

| | | | | |
|---|---|---|---|---|
| 1 | **GGGCGAAUUG** | **GGCCCUCUAG** | **AUGCAUG**CUC | GAGUAAAUAG |
| 41 | CCCUCACCCA | CCAACUAGCC | GUUACAGGCA | AGUUACUGCG |
| 81 | CGAUGGCGCA | CCGGACAGUC | CGGUGCGCCA | CCGGUGCGCC |
| 121 | ACCGGUGCGC | CACCGGUGCG | CCAACGGUCA | CUUNCAACGG |
| 161 | CUAGUUCUGA | CACAGAGCCG | UUGGACUCAU | GACGCACCGG |
| 201 | ACAGUGAAUA | GUUCACUGUC | CGGUGCACAC | CGGACAGUCC |
| 241 | GGUGCGGUGU | CCGGUGUGCC | ACUAAAAUUC | AUCUCCGAAG |
| 281 | CCUGCGCUCU | CGGACUAGU**G** | **CAUCCGAGCU** | **CGGUACCA** |

## EXPERIMENT 9

## USE OF MET IC IN CT, GC, AND HCV ASSAYS

Independent RT-PCR assays were carried out with TAQMAN® probes (Applied Biosystems, Foster City, CA) for CT, GC, and HCV, respectively. Each assay included the target nucleic acid (DNA for CT and GC and RNA for HCV) and the MET IC of the present invention in a single well. Figures 2, 3, and 4 show the results of the amplification assays (cycle number versus delta Rn). Rn, the normalized reporter signal, is the fluorescence signal of the reporter dye divided by the fluorescence signal of the internal reference dye. Delta Rn (dRn) is determined by the formula $R^{n+} - R^{n-}$, where $R^{n+}$ is the Rn value for a reaction involving all components and $R^{n-}$ is the value for an unreacted sample. In each graph, the curve on the left represents amplification of the target and the curve on the right represents amplification of the IC.

## Claims

1. The use of a non-competitive internal control for use in nucleic acid tests (NATs), said non-competitive internal control comprising a nucleic acid obtained from *Methanobacterium thermoautrophicum* (MET).

2. The use of a non-competitive internal control of claim 1, wherein the nucleic acid is DNA.

3. The use of a non-competitive internal control of claim 2, wherein the NATs are selected from the group consisting of: Influenza A, Influenza B, parainfluenza viruses 1 to 4 (PIV-1 to PIV-4), respiratory syncytial virus type A (RSV A), RSV B, human metapneumovirus (hMPV), Hepatitis B virus (HBV), *Chlamydia trachomatis* (CT), and *Neisseria gonorrhea* (GC).

4. The use of a non-competitive internal control of claim 1, wherein the nucleic acid is RNA.

5. The use of a non-competitive internal control of claim 4, wherein the diagnostic NATs are selected from the group consisting of Hepatitis C virus (HCV), Human Immunodeficiency Virus 1 (HIV-1), and Severe Acute Respiratory Syndrome (SARS).

6. The use of a non-competitive internal control of claim 1, wherein the nucleic acid comprises at least at least two primer binding sites and at least one probe binding site.

7. A method of preparing a non-competitive internal control for use in.nucleic acid tests (NATs), comprising the steps of:

   (a) extracting genomic DNA from *Methanobacterium thermoautrophicum* (MET);
   (b) generating an amplicon from the genomic DNA of step (a) using forward and reverse primers having at least two restriction enzyme sites and a target specific sequence;
   (c) generating a plasmid by ligating the amplicon of step (b) with a vector sequence having a promoter sequence and restriction enzyme sites that are identical to the restriction enzyme sites of the amplicon;
   (d) digesting the plasmid with restriction enzymes corresponding to the restriction enzyme sites of steps (b) and (c) to generate MET internal control DNA.

8. The method of claim 7, further comprising the step of:

   (e) preparing MET internal control RNA from the DNA of step (e).

9. The method of claim 7, wherein the at least two restriction enzyme sites of steps (b) and (d) correspond to the sequences of restriction enzymes XhoI and SpeI.

10. The method of claim 7, wherein the forward primer of step (b) has the sequence of SEQ ID. NO. 3 and the reverse primer of step (b) has the sequence of SEQ ID. NO. 4.

11. The method of claim 7, wherein the promoter sequence of step (c) is a T7 promoter sequence.

12. The method of claim 7, wherein the MET internal control DNA is used as an internal control in DNA nucleic acid diagnostic tests for the following disease states: Influenza A, Influenza B, parainfluenza viruses 1 to 4 (PIV-1 to PIV-4), respiratory syncytial virus type A (RSV A), RSV B, human metapneumovirus (hMPV), Hepatitis B virus (HBV), *Chlamydia trachomatis* (CT), and *Neisseria gonorrhea* (GC).

13. The method of claim 8, wherein the MET internal control RNA is used as an internal control in RNA nucleic acid diagnostic tests for the following disease states: Hepatitis C virus (HCV), Human Immunodeficiency Virus I (HIV-1), and Severe Acute Respiratory Syndrome (SARS).

14. A non-competitive internal control for use in nucleic acid tests (NATs), comprising a nucleic acid obtained from *Methanobacterium thermoautrophicum* (MET)according to any one of SEQ ID NO: 8, 10, 12, 14, 18, 22, 26 37, 38, 39, and 40.

**Patentansprüche**

1. Verwendung einer nichtkompetitiven internen Kontrolle zur Verwendung in Nukleinsäuretests (NATs), wobei die nichtkompetitive interne Kontrolle eine Nukleinsäure aus *Methanobacterium thermoautrophicum* (MET) umfasst.

2. Verwendung einer nichtkompetitiven internen Kontrolle nach Anspruch 1, wobei es sich bei der Nukleinsäure um DNA handelt.

3. Verwendung einer nichtkompetitiven internen Kontrolle nach Anspruch 2, wobei die NATs aus der Gruppe bestehend aus den Folgenden ausgewählt sind: Influenza A, Influenza B, Parainfluenzaviren 1 bis 4 (PIV-1 bis PIV-4), Respiratorisches Synzytial-Virus Typ A (RSV A), RSV B, humanes Metapneumovirus (hMPV), Hepatitis-B-Virus (HBV), *Chlamydia trachomatis* (CT) und *Neisseria gonorrhea* (GC).

4. Verwendung einer nichtkompetitiven internen Kontrolle nach Anspruch 1, wobei es sich bei der Nukleinsäure um RNA handelt.

5. Verwendung einer nichtkompetitiven internen Kontrolle nach Anspruch 4, wobei die diagnostischen NATs aus der Gruppe bestehend aus Hepatitis-C-Virus (HCV), menschlichem Immundefizienzvirus 1 (HIV-1) und Schwerem Akutem Atemwegssyndrom (SARS) ausgewählt sind.

6. Verwendung einer nichtkompetitiven internen Kontrolle nach Anspruch 1, wobei die Nukleinsäure mindestens zwei Primer-Bindungsstellen und mindestens eine Sondenbindungsstelle umfasst.

7. Verfahren zur Herstellung einer nichtkompetitiven internen Kontrolle zur Verwendung in Nukleinsäuretests (NATs), umfassend die folgenden Schritte:

   (a) Extrahieren von genomischer DNA aus *Methanobacterium thermoautrophicum* (MET);
   (b) Erzeugen eines Amplicons aus der genomischen DNA von Schritt (a) unter Einsatz von Forward- und Reverse-Primern mit mindestens zwei Restriktionsenzymspaltstellen und einer zielspezifischen Sequenz;
   (c) Erzeugen eines Plasmids durch Ligieren des Amplicons aus Schritt (b) mit einer Vektorsequenz, die eine Promotersequenz und Restriktionsenzymspaltstellen, die mit den Restriktionsenzymspaltstellen des Amplicons identisch sind, aufweist;
   (d) Verdauen des Plasmids mit Restriktionsenzymen, die den Restriktionsenzymspaltstellen der Schritte (b) und (c) entsprechen, wodurch eine interne Kontroll-MET-DNA entsteht.

8. Verfahren nach Anspruch 7, das weiterhin den folgenden Schritt umfasst:

   (e) Herstellen einer internen Kontroll-MET-RNA aus der DNA von Schritt (e).

9. Verfahren nach Anspruch 7, wobei die mindestens zwei Restriktionsenzymspaltstellen der Schritte (b) und (d) den Sequenzen der Restriktionsenzyme XhoI und SpeI entsprechen.

10. Verfahren nach Anspruch 7, wobei der Forward-Primer von Schritt (b) die Sequenz von SEQ ID NO: 3 und der Reverse-Primer von Schritt (b) die Sequenz von SEQ ID NO: 4 aufweist.

11. Verfahren nach Anspruch 7, wobei es sich bei der Promotersequenz von Schritt (c) um eine T7-Promotersequenz handelt.

12. Verfahren nach Anspruch 7, wobei die interne Kontroll-MET-DNA als interne Kontrolle in diagnostischen DNA-Nukleinsäuretests für die folgenden Krankheiten verwendet wird: Influenza A, Influenza B, Parainfluenzaviren 1 bis 4 (PIV-1 bis PIV-4), Respiratorisches Synzytial-Virus Typ A (RSV A), RSV B, humanes Metapneumovirus (hMPV), Heptatitis-B-Virus (HBV), *Chlamydia trachomatis* (CT) und *Neisseria gonorrhea* (GC).

13. Verfahren nach Anspruch 8, wobei die interne Kontroll-MET-RNA als interne Kontrolle in diagnostischen RNA-Nukleinsäuretests für die folgenden Krankheiten verwendet wird: Hepatitis-C-Virus (HCV), menschliches Immundefizienzvirus 1 (HIV-1) und Schweres Akutes Atemwegssyndrom (SARS).

14. Nichtkompetitive interne Kontrolle zur Verwendung in Nukleinsäuretests (NATs), umfassend eine aus *Methanob-*

*acterium thermoautrophicum* (MET) nach einem der SEQ ID NO: 8, 10, 12, 14, 18, 22, 26, 37, 38, 39 und 40 erhaltene Nukleinsäure.

## Revendications

**1.** Utilisation d'un témoin interne non compétitif à utiliser dans des essais d'acide nucléique (EANs), le témoin interne non compétitif comprenant un acide nucléique obtenu à partir de *méthanobactérium thermoautrophicum* (MET).

**2.** Utilisation d'un témoin interne non compétitif suivant la revendication 1, dans lequel l'acide nucléique est l'ADN.

**3.** Utilisation d'un témoin interne non compétitif suivant la revendication 2, dans lequel les EANs sont choisis dans le groupe consistant en : influenza A, influenza B, virus de parainfluenza 1 à 4 (PIV-1 à PIV-4), virus syncytial respiratoire de type A (RSV A), RSV B, métapneumovirus humain (hMPV), virus de l'hépatite B (HBV), *chlamydia trachomatis* (CT) et *neisseria gonorrhea* (GC).

**4.** Utilisation d'un témoin interne non compétitif suivant la revendication 1, dans lequel l'acide nucléique est l'ARN.

**5.** Utilisation d'un témoin interne non compétitif suivant la revendication 4, dans lequel les EANs de diagnostic sont choisis dans le groupe consistant en le virus de l'hépatite C (HCV), en le virus d'immunodéficience humain 1 (VIH-1) et le syndrome respiratoire aigu sévère (SARS).

**6.** Utilisation d'un témoin interne non compétitif suivant la revendication 1, dans lequel l'acide nucléique comprend au moins deux sites de liaison d'amorce et au moins un site de liaison de sonde.

**7.** Procédé de préparation d'un témoin interne non compétitif à utiliser dans des essais d'acide nucléique (EANs), comprenant les stades dans lesquels :

(a) on extrait de l'ADN génomique de *méthanobactérium thermoautrophicum* (MET);
(b) on produit un amplicon à partir de l'ADN génomique du stade (a), en utilisant des amorces sens et anti-sens ayant au moins deux sites d'enzyme de restriction et une section spécifique cible ;
(c) on produit un plasmide en ligaturant l'amplicon du stade (b) avec une séquence de vecteur ayant une séquence de promoteur et des sites d'enzyme de restriction qui sont identiques aux sites d'enzyme de restriction de l'amplicon ;
(d) on fait digérer le plasmide par des enzymes de restriction correspondants aux sites d'enzyme de restriction des stades (b) et (c) pour produire le témoin interne MET d'ADN.

**8.** Procédé suivant la revendication 7, comprenant en outre le stade dans lequel

(e) on prépare le témoin interne MET d'ARN à partir de l'ADN du stade (e).

**9.** Procédé suivant la revendication 7, dans lequel les au moins deux sites d'enzyme de restriction des stades (b) et (d) correspondent aux séquences des enzymes de restriction XhoI et SpeI.

**10.** Procédé suivant la revendication 7, dans lequel l'amorce sens du stade (b) a la séquence de l'identification de séquence n°3 et l'amorce anti-sens du stade (b) a la séquence de l'identification de séquence n°4.

**11.** Procédé suivant la revendication 7, dans lequel la séquence de promoteur de stade (c) est une séquence de promoteur T7.

**12.** Procédé suivant la revendication 7, dans lequel le témoin interne MET d'ADN est utilisé dans un témoin interne dans des essais de diagnostic d'acide nucléique d'ADN pour les états maladifs suivants : influenza A, influenza B, virus de parainfluenza 1 à 4 (PIV-1 à PIV-4), virus syncytial respiratoire de type A (RSV A), RSV B, métapneumovirus humain (hMPV), virus de l'hépatite B (HBV), *chlamydia trachomatis* (CT) et *neisseria gonorrhea* (GC).

**13.** Procédé suivant la revendication 8, dans lequel le témoin interne MET d'ARN est utilisé comme un témoin interne dans des tests de diagnostic d'acide nucléique d'ARN pour les états maladifs suivants : virus de l'hépatite C (HCV), virus d'immunodéficience humain 1 (VIH-1) et le syndrome respiratoire aigu sévère (SARS).

**14.** Témoin interne non compétitif à utiliser dans des essais d'acide nucléique (EANs), comprenant un acide nucléique obtenu à partir de *méthanobactérium thermoautrophicum* (MET) suivant l'une quelconque des identifications de séquences n° 8, 10, 12, 14, 18, 22, 26, 37, 38, 39 et 40.

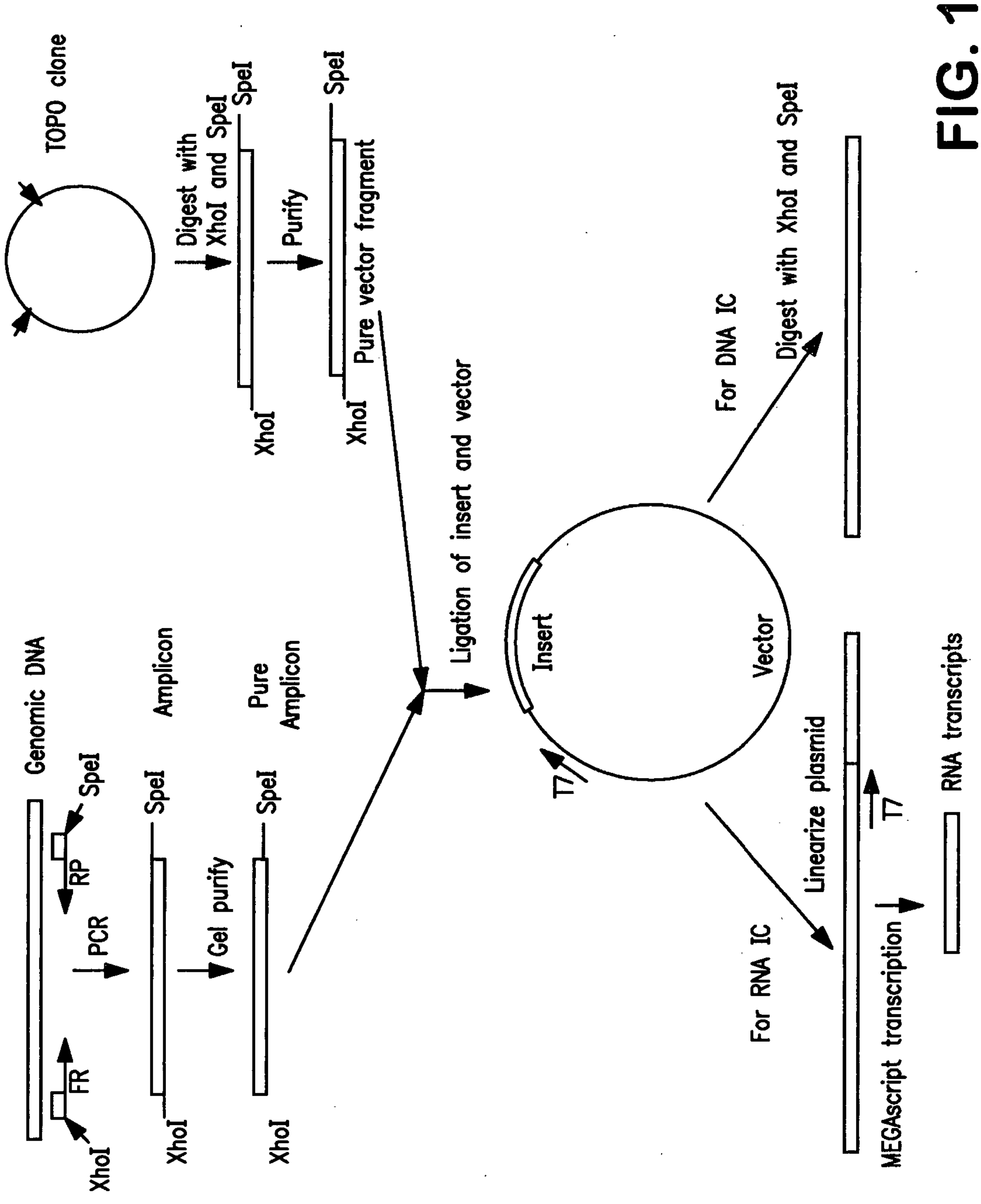
Genomic DNA
XhoI
FR
RP
SpeI
PCR
Amplicon
XhoI
SpeI
Gel purify
Pure Amplicon
XhoI
SpeI
TOPO clone
Digest with XhoI and SpeI
XhoI
SpeI
Purify
XhoI
SpeI
Pure vector fragment
Ligation of insert and vector
Insert
Vector
T7
For DNA IC
Digest with XhoI and SpeI
For RNA IC
Linearize plasmid
T7
MEGAscript transcription
RNA transcripts

FIG. 1

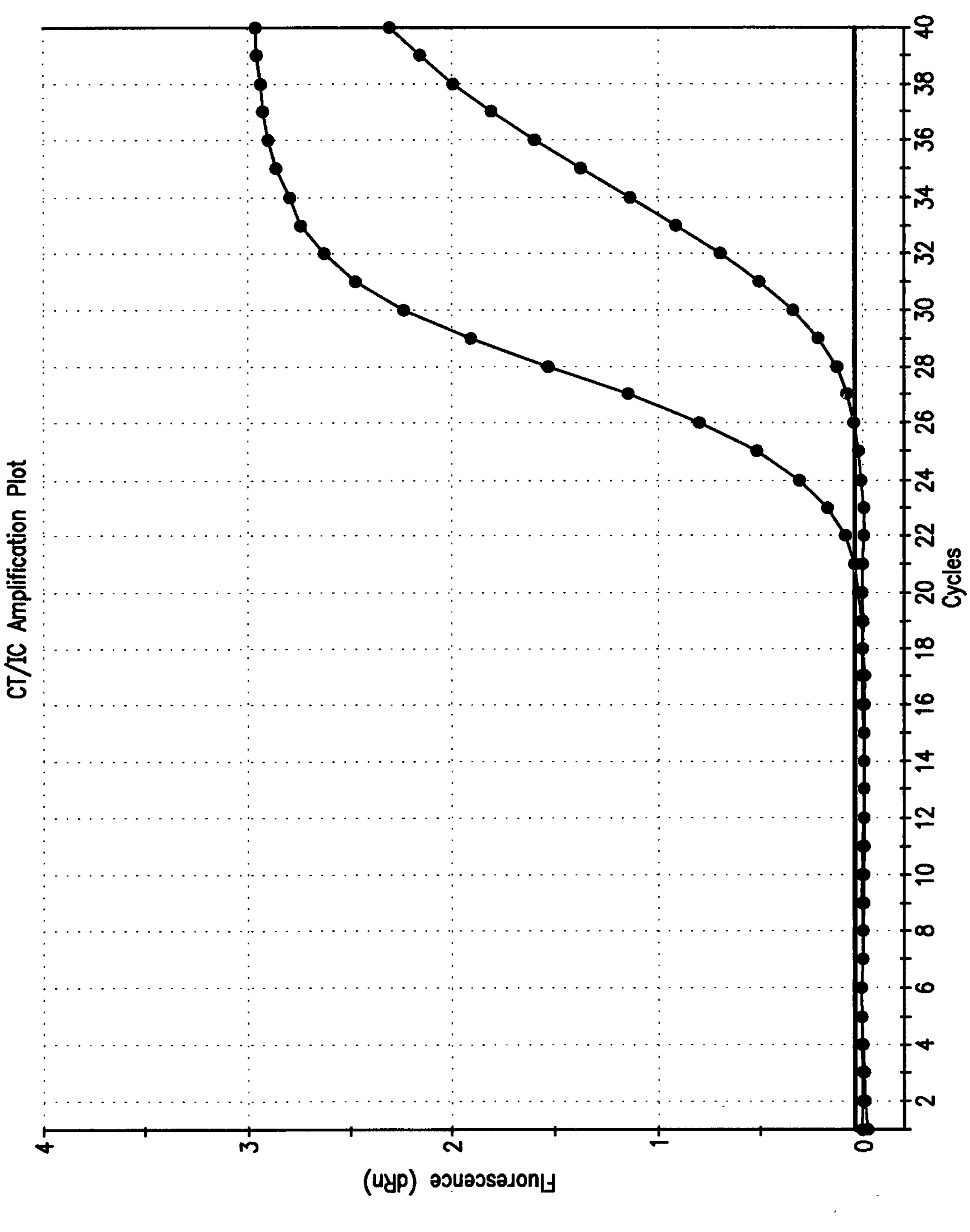
CT/IC Amplification Plot
Fluorescence (dRn)
Cycles
0
1
2
3
4
2
4
6
8
10
12
14
16
18
20
22
24
26
28
30
32
34
36
38
40

FIG. 2

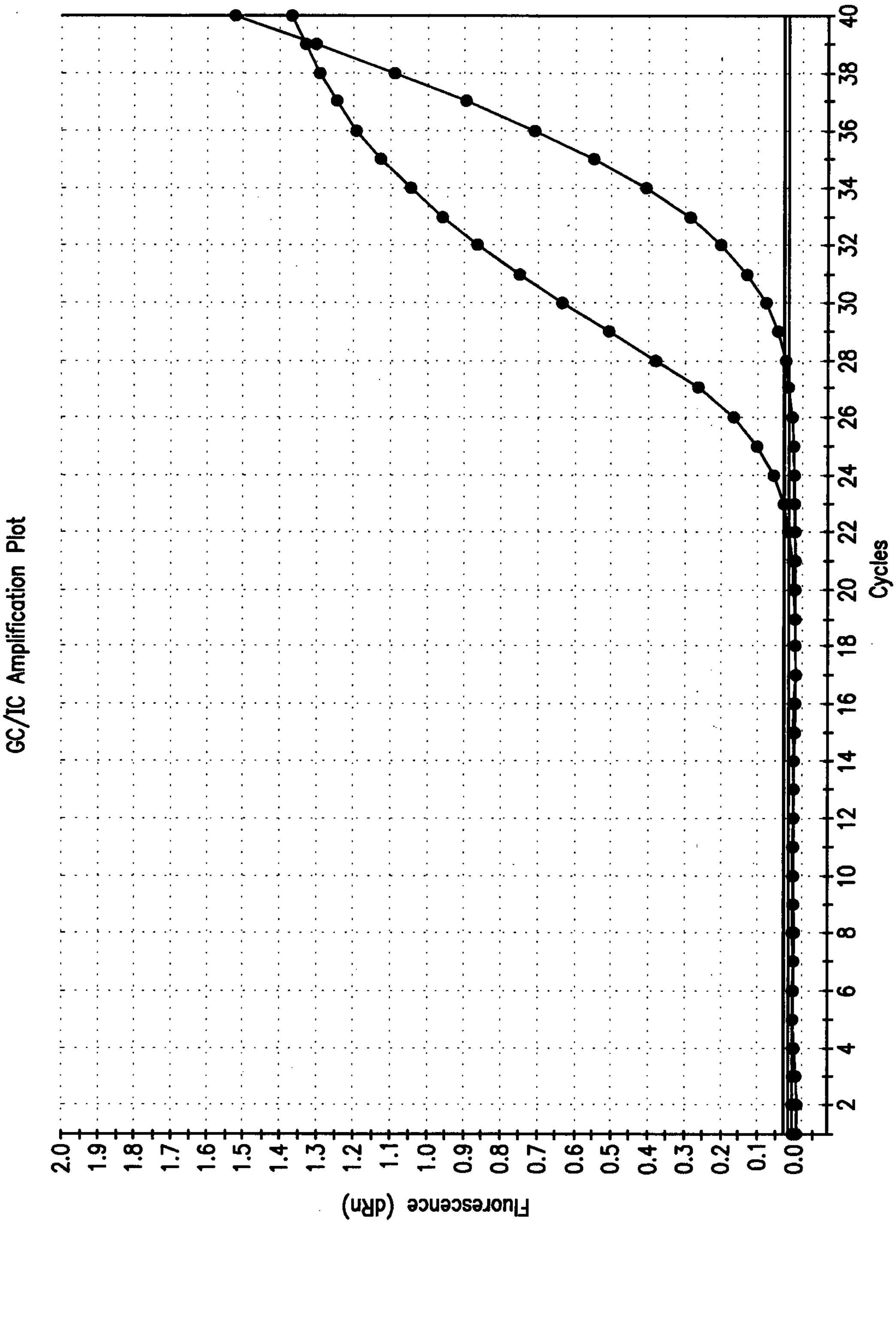
GC/IC Amplification Plot
Fluorescence (dRn)
2.0 1.9 1.8 1.7 1.6 1.5 1.4 1.3 1.2 1.1 1.0 0.9 0.8 0.7 0.6 0.5 0.4 0.3 0.2 0.1 0.0
Cycles
2 4 6 8 10 12 14 16 18 20 22 24 26 28 30 32 34 36 38 40

FIG. 3

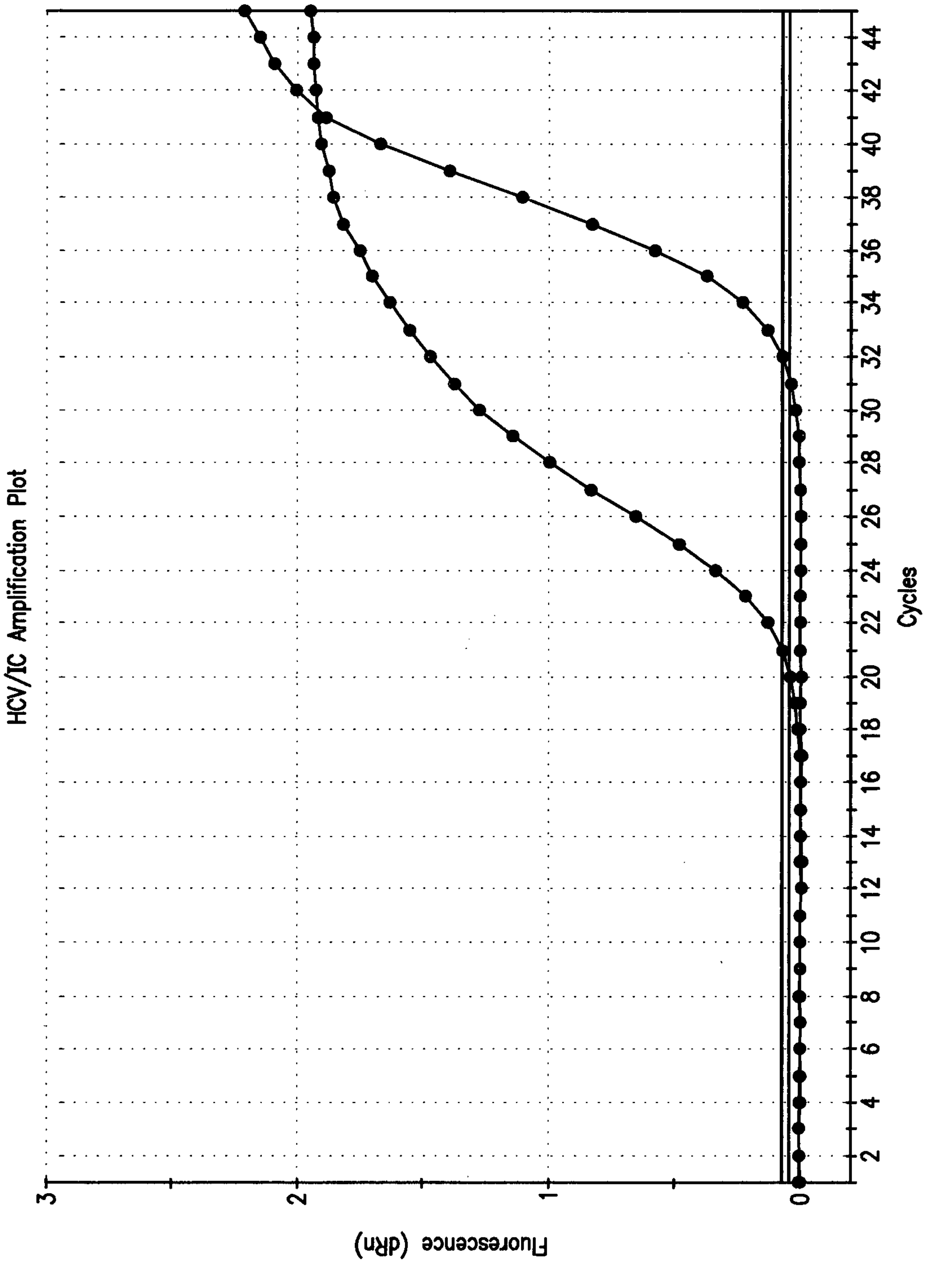
HCV/IC Amplification Plot
Fluorescence (dRn)
0
1
2
3
Cycles
2 4 6 8 10 12 14 16 18 20 22 24 26 28 30 32 34 36 38 40 42 44

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4683195 A, Mullins
- US 4683202 A, Mullis
- WO 61038064 A


### Non-patent literature cited in the description


- Oevelopment of a novel internal positive control for Taqman (R) based assays. **HARTMAN L J et al.** MOLECULAR ANO CELLULAR PROBES. ACAOEMIC PRESS, 01 February 2005, vol. 19, 51-59
- **HARTMAN et al.** *Molecular and Cellular Probes,* 2005, vol. 19, 51-59